# EUROPEAN PATENT SPECIFICATION

(11) **EP 3 530 320 B1**
(45) Date of publication and mention of the grant of the patent: **15.11.2023**
(21) Application number: 18158758.5
(22) Date of filing: 27.02.2018
(51) Int. Cl.: A61P 25/28, C07K 14/54

(54) **NOVEL IL-4-/IL-13-DERIVED PEPTIDE COMPOUNDS FOR THE TREATMENT OR PREVENTION OF NEURODEGENERATIVE OR NEUROINFLAMMATORY DISEASES**
NEUARTIGE IL-4-/IL-13-ABGELEITETE PEPTIDVERBINDUNGEN ZUR BEHANDLUNG ODER PRÄVENTION VON NEURODEGENERATIVEN ODER NEUROINFLAMMATORISCHEN ERKRANKUNGEN
NOUVEAUX COMPOSÉS PEPTIDIQUES DÉRIVÉS IL-4-/IL-13 POUR LE TRAITEMENT OU LA PRÉVENTION DE MALADIES NEURODÉGÉNÉRATIVES OU NEURO-INFLAMMATOIRES

(43) Date of publication of application: 28.08.2019
(73) Proprietor: Universitätsmedizin der Johannes Gutenberg-Universität Mainz, 55131 Mainz (DE)
(72) Inventor: VOGELAAR, Christina Francisca, 55128 Mainz (DE); ZIPP, Frauke, 60323 Frankfurt (DE)
(74) Representative: Patentanwälte Dr. Keller, Schwertfeger Partnerschaft mbB

(56) References cited:
- WO-A2-2006/036878
- MUELLER T D ET AL: "Structure, binding, and antagonists in the IL-4/IL-13 receptor system", BIOCHIMICA ET BIOPHYSICA ACTA. MOLECULAR CELL RESEA, ELSEVIER SCIENCE PUBLISHERS, AMSTERDAM, NL, vol. 1592, no. 3, 11 November 2002 (2002-11-11), pages 237-250, XP004391603, ISSN: 0167-4889, DOI: 10.1016/S0167-4889(02)00318-X
- MOY F J ET AL: "Solution structure of human IL-13 and implication for receptor binding", JOURNAL OF MOLECULAR BIO, ACADEMIC PRESS, UNITED KINGDOM, vol. 310, no. 1, 29 June 2001 (2001-06-29) , pages 219-230, XP004464193, ISSN: 0022-2836, DOI: 10.1006/JMBI.2001.4764
- ZUEGG J ET AL: "Structural model of human IL-13 defines the spatial interactions with the IL-13Ralpha/IL-4Ralpha receptor", IMMUNOLOGY AND CELL BIOLOGY, CARLTON, AU, vol. 79, no. 4, 1 August 2001 (2001-08-01) , pages 332-339, XP002304540, ISSN: 0818-9641, DOI: 10.1046/J.1440-1711.2001.01035.X
- SIMONE MORI ET AL: "Neuroimmunology of the Interleukins 13 and 4", BRAIN SCIENCES, vol. 6, no. 2, 13 June 2016 (2016-06-13), page 18, XP055497696, DOI: 10.3390/brainsci6020018

## Description

The present invention relates to novel peptide compounds that are derived from human or animal interleukin-4 (IL-4) or interleukin-13 (IL-13) and the uses in a treatment or prevention of neuroinflammatory or neurodegenerative disorders, neuropathies or traumatic nervous system injuries.

Ongoing and accumulating axon pathology is considered the main feature underlying disability, especially during the progressive disease phase of neurodegenerative disorders (V. Siffrin, H. Radbruch, R. Glumm, R. Niesner, M. Paterka, J. Herz, T. Leuenberger, S. M. Lehmann, S. Luenstedt, J. L. Rinnenthal, G. Laube, H. Luche, S. Lehnhardt, H. J. Fehling, O. Griesbeck, F. Zipp, In vivo imaging of partially reversible th17 cell-induced neuronal dysfunction in the course of encephalomyelitis. Immunity 33, 424-436 (2010); I. Nikic, D. Merkler, C. Sorbara, M. Brinkoetter, M. Kreutzfeld, F. M. Bareyre, W. Bruck, D. Bishop, T. Misgeld, M. Kerschensteiner, A reversible form of axon damage in experimental autoimmune encephalomyelitis and multiple sclerosis. Nat. Med. 17, 495-499 (2011)). Upon an inflammatory attack, axonal swelling and persisting upregulation of calcium eventually culminate in beading and degeneration These processes of inflammatory neuronal injury can at least partly be reversed. To date, the axon compartment has not been sufficiently targeted by MS treatment strategies (C. Larochelle, T. Uphaus, A. Prat, F. Zipp, Secondary Progression in Multiple Sclerosis: Neuronal Exhaustion or Distinct Pathology? Trends Neurosci. 39, 325-339 (2016); R. J. M. Franklin, C. ffrench-Constant, J. M. Edgar, K. J. Smith, Neuroprotection and repair in multiple sclerosis. Nat. Rev. Neurol. 8, 624-634 (2012), F. Zipp, R. Gold, H. Wiendl, Identification of inflammatory neuronal injury and prevention of neuronal damage in multiple sclerosis: hope for novel therapies? JAMA Neurol 70, 1569-1574 (2013)).

Interleukin-4 (IL-4) and interleukin-13 (IL-13) are canonical type 2 cytokines that play overlapping but distinct roles in mammalian immune responses to extracellular parasites by stimulating production of high affinity IgE antibodies, the generation of alternatively activated macrophages and the differentiation of Th2 cells. IL-4 is an anti-inflammatory cytokine with respect to effects on the central nervous system (CNS), but systemic IL-4 plays a role in allergies, in particular asthma. IL-4 has been reported to have beneficial effects on neurons upon traumatic CNS injury (J. T. Walsh, S. Hendrix, F. Boato, I. Smirnov, J. Zheng, J. R. Lukens, S. Gadani, D. Hechler, G. Golz, K. Rosenberger, T. Kammertons, J. Vogt, C. Vogelaar, V. Siffrin, A. Radjavi, A. Fernandez-Castaneda, A. Gaultier, R. Gold, T. D. Kanneganti, R. Nitsch, F. Zipp, J. Kipnis, MHCII-independent CD4+ T cells protect injured CNS neurons via IL-4. J. Clin. Invest. 125, 699-714 (2015)). Both an immune regulatory function of systematically applied IL-4 in experimental autoimmune encephalomyelitis (EAE), the murine model of multiple sclerosis (MS), and a destructive role in an asthma model have been reported (N. L. Payne, A. Dantanarayana, G. Sun, L. Moussa, S. Caine, C. McDonald, D. Herszfeld, C. C. Bernard, C. Siatskas, Early intervention with gene-modified mesenchymal stem cells overexpressing interleukin-4 enhances anti-inflammatory responses and functional recovery in experimental autoimmune demyelination. Cell Adh. Migr. 6, 179-189 (2012); M. K. Racke, A. Bonomo, D. E. Scott, B. Cannella, A. Levine, C. S. Raine, E. M. Shevach, M. Rocken, Cytokine-induced immune deviation as a therapy for inflammatory autoimmune disease. J. Exp. Med. 180, 1961-1966 (1994); J. I. Inobe, Y. Chen, H. L. Weiner, In vivo administration of IL-4 induces TGF-beta-producing cells and protects animals from experimental autoimmune encephalomyelitis. Ann. N. Y. Acad. Sci. 778, 390-392 (1996); S. T. Holgate, Innate and adaptive immune responses in asthma. Nat. Med. 18, 673-683 (2012); C. M. Lloyd, E. M. Hessel, Functions of T cells in asthma: more than just T(H)2 cells. Nat. Rev. Immunol. 10, 838-848 (2010)).

It is known that IL-4 potentially has side effects due to its effects on the immune system (S. T. Holgate, Innate and adaptive immune responses in asthma. Nat Med 18, 673-683 (2012); C. M. Lloyd, and E. M. Hessel, Functions of T-cells in asthma: more than just T(H)2 cells. Nat Rev Immunol 10, 838-848 (2010)). Furthermore, it would be extremely expensive to produce large amounts of recombinant IL-4 for clinical purposes in humans. In addition, IL-4 has a very short half-life in blood plasma (S. Gea-Sorli, and D. Closa, In vitro, but not in vivo, reversibility of peritoneal macrophages activation during experimental acute pancreatitis. BMC Immunol 10, 42 (2009); M. Khodoun, C. C. Lewis, J. Q. Yang, T. Orekov, C. Potter, T. Wynn, M. Mentink-Kane, G. K. Hershey, M. Wills-Karp, and F. D. Finkelman, Differences in expression, affinity, and function of soluble (s)IL-4Rα and sIL-13Rα2 suggest opposite effects on allergic responses. J Immunol 179, 6429-6438 (2007)). It is therefore desirable to be in possession of compounds that could be produced cost-effectively in large amounts and that would be more stable than recombinant full-length IL-4.

Whilst their amino acid identity is low (23%), IL-4 and IL-13 can bind to a common receptor composed of the IL-4Ra and IL-13Rα1 subunits (type II IL-4R) and they are the only cytokines known to bind to the receptor chain IL-4Rα. In addition, IL-4 can bind to the IL-4R type I, which is composed of IL-4Rα and the common γ chain (T. Wang, and C. J. Secombes, The evolution of IL-4 and IL-13 and their receptor subunits. Cytokine 75, 8-13 (2015)). For signal transduction, both IL-4 and IL-13 require the same receptor sub-unit, IL-4Ra (T. D. Mueller, J. L. Zhang, W. Sebald, A. Duschl, Structure, binding and antagonists in the IL-4/IL-13 receptor system, Biochimica Biophysica Acta 1592, 237-250 (2002)). IL-4Ra is part of both functional heterodimeric receptor complexes. It organizes signal transduction through signalling molecules associated with its large intracellular domain. IL-4 and IL-13 have an identical dimeric receptor subunit assembly characterized by antiparallel juxtaposed helices αA, αC, αB, αD and two long end-to-end loops, loop AB and CD, which are connected by a short β-sheet packed against helices B and D. Helix A, the AB loop, the helix BC hairpin and the loop CD plus helix D are encoded by four different axons. Multiple alignments of the aa sequences of selected IL-4, IL-13 and IL-4/13 proteins reveal that in general four cysteine residues are present in each protein but the patterns of the cysteine residues are lineage-specific, i.e. mammalian IL-4 and IL-13, teleost fish IL-4/13, and other vertebrate IL-4/13 (T. Wang, and C. J Secombes, The evolution of IL-4 and IL-13 and their receptor subunits. Cytokine 75, 8-13 (2015); F. J. Moy et al., Solution structure of human IL-13 and implication for receptor binding. Journal of Molecular Bio 310, 219-230 (2001); J. Zuegg et al., Structural model of human IL-13 defines the spatial interactions with the IL-13Ralpha/lL-4Ralpha receptor. Immunology and cell biology 79, 332-339 (2001)).

The IL-4 and IL-13 cytokines are dominated by a 4α-helix bundle with a left-handed twist (M. L. Walter, W. J. Cook, B. G. Zhao, R. P. Cameron Jr, S. E. Ealick, R. L. Walter Jr, P. Reichert, T. L. Nagabhushan, P.P Trotta, C. E. Bugg, Crystal structure of recombinant human interleukin-4. J Biol Chem 267, 20371-6 (1992)). The secondary structural features of IL-13 are similar to that of IL-4. Similarly, IL-13 contains 4α-helical bundles (E. L. Rael, R. F. Lockey, Interleukin-13 signaling and its role in asthma. World Allergy Organ J 4, 54-64 (2011)).

Both the IL-4 and IL-13 cytokines share a common heterodimeric receptor. Both can downregulate the synthesis of T-helper type 1 pro-inflammatory cytokines. Both neuroprotective or neurotoxic effects have been proposed based on evidence that interleukin 13 and 4 can reduce inflammation by promoting the M2 microglia phenotype and contributing to the death of microglia M1 phenotype, or by potentiating the effects of oxidative stress on neurons during neuro-inflammation (S. Mori et al., Neuroimmunology of the Interleukins 13 and 4. Brain sciences 6, 18 (2016).

WO 2006/036878 A2 discloses chimeric polypeptide antagonists that include an interleukin-4 mutein linked to an IL-9 mutein to reduce or inhibit the responsiveness of a cell to IL-4, IL-9 and/or IL-13.

Given the role of IL-4/IL-13 in immune responses and neuroinflammatory and neurodegenerative disorders, the possession of agents that are able to bind to IL-4R type I and/or type II and to ameliorate clinical signs, would be highly desirable. The patent US 5,017,691 describes IL-4 polypeptides, which were tested for IL-4 activity on B-cell and T-cell growth. Native human and murine IL-4 proteins and muteins thereof, and their cDNAs coding for mammalian IL-4s and their muteins are described. However, these IL-4 polypeptides cause undesired immune responses that are not wanted for the treatment of neurodegenerative disorders.

EP 2365 983 A1 describes IL-4-derived peptides for modulation of a chronic inflammatory response and treatment of autoimmune diseases. The peptide comprises at most 35 contiguous amino acids, which are derived from an α-helix of IL-4, each peptide consisting of the amino acids AQFHRHKQLIRFLKRA.

Furthermore, novel synthetic peptides, termed Ph8, derived from the α-helix C of IL-4, which interacts with IL-4 receptor α (IL-4 Rα), have been described and it was found that Ph-bound IL-4 Rα mimicked the anti-inflammatory effects of IL-4 by inhibiting TNF-α production by macrophages *in vitro.* Ph8 inhibited the proliferation of Th1/2 cells and downregulated the production of EFN-γ in stimulated Th1 cells. It was also found that Ph8 did not induce general T-cell activation and inflammatory responses without further inducing the side effects generally associated with IL-4 signalling (B. Klementiev, M. N. Enevoldsen, S. Li, R. Carlsson, Y. Liu, S. Issazadeh-Navikas, E. Bock, V. Berezin, Antiinflammatory properties of a peptide derived from interleukin-4. Cytokine 64, 112-121 (2013)). Ph8 peptides, however, are not suitable for neuroprotective or neuroregenerative purposes as the peptides have an effect on lymphocytes and bone marrow-derived macrophages.

The inventors surprisingly discovered that IL-4 could ameliorate clinical symptoms in mice subjected to experimental autoimmune encephalomyelitis (EAE), a mouse model of multiple sclerosis (MS), when applied intrathecally during the chronic phase of the disease. The effect of IL-4 and the inventive derivatives of IL-4 was accompanied by improved axonal morphology as well as increased regenerative sprouting. Using *in vitro* models, it has been observed by the inventors that IL-4 plays a role in neuroprotection, axon regeneration and growth on inhibitory substrates. In particular, it was discovered that IL-4 effects are abolished in neuron-specific IL-4 receptor (IL-4R) knock-out mice. A fast direct IL-4R signalling pathway has been identified. In addition, the nasal application IL-4 was equally effective, providing a more clinically relevant route of administration for the treatment of neuronal disorders (C. F. Vogelaar, S. Mandal, S. Lerch, K. Birkner, J. Birkenstock, U. Bühler, A. Schnatz, C. S. Raine, S. Bittner, J. Vogt, J. Kipnis, R. Nitsch, F. Zipp, Fast direct neuronal signaling via the IL-4 receptor as therapeutic target in neuroinflammation. Sci Transl Med doi: 10.1 126/scitranslmed.aao2304 (2018)).

The role of IL-4 in neuroinflammation, combined with the neuroprotective role in traumatic injury and the identified neuronal signalling pathway suggests that IL-4 and its IL-4 derivatives and IL-13 derivatives are suitable for the treatment or prevention of neuroinflammatory and neurodegenerative diseases, in particular for the prevention or treatment of multiple sclerosis (MS).

Against this background, it is the object of the present invention to provide alternative compounds that act positively on neurons, have no side effect on lymphocytes or bone marrow-derived macrophages and are suitable for the prevention or treatment of neuronal disorders.

This object is solved by the claimed compounds and their uses in the prevention or treatment of neuroinflammatory or neurodegenerative disorders, neuropathies or traumatic nervous system injuries. Preferred embodiments of the invention are claimed in the sub-claims.

Due to the high structural similarity of the secondary structural features of IL-4 and IL-13 and the fact that both cytokines contain 4α-helical bundles, herein designated with the letters αA, αD, αC, αD, the inventive compounds have in common that the crucial amino acids are derived from the αA to αD alpha helical regions of IL-4 and IL-13, respectively.

The term "IL-4 derivative" as used in the context of the present invention designates a compound, preferably a peptide compound, that is based on native IL-4. Preferably, a IL-4 derivative of the present invention has an identical or highly similar amino acid sequence and/or structure as at least three selected domains from the relevant αA to αD alpha helical binding regions of IL-4.

The term "IL-13 derivative" as used in the context of the present invention designates a compound, preferably a peptide compound, that is based on native IL-13. Preferably, a IL-13 derivative of the present invention has an identical or highly similar amino acid sequence and/or structure as at least three selected domains from the relevant αA to αD alpha helical binding regions of IL-13.

The compounds of the present invention consist of one or more peptides that are derived from the αA to αD alpha helical regions of IL-4 or IL-13 comprising the following general structure:

A - L1 - B - L2 - C

wherein
A corresponds to a first amino acid sequence that is derived from αA or αC alpha helical region of human or animal IL-4 or IL-13,
B corresponds to a second amino acid sequence that is derived from αA or αC alpha helical region of human or animal IL-4 or IL-13,
C corresponds to a third amino acid sequence that is derived from αD or αB alpha helical region of human or animal IL-4, or αD alpha helical region of human or animal IL-13,
L1 and L2 correspond to one or more linking amino acids

The term "alpha-helix" (α-helix) designates a common motif in the secondary structure of the peptides. The α-helix can be in a right- or left-handed coiled conformation, in which every backbone N-H group donates a hydrogen bond to the backbone C=O group of the amino acid four residues earlier.

Accordingly, one or more amino acid sequences of the αA to αD alpha helical regions of IL-4 or IL-13 may be selected to form the parts A, B and C of the peptides of the present invention. Preferably, the selected amino acids are contiguous amino acids that are found in the corresponding α-helical regions of human or animal IL-4 or IL-13, or variants thereof in which one or more amino acids in a given amino acid sequence are deleted, added, substituted or modified. Preferred peptides of the invention comprise amino acid sequences derived from the αA-αC-αD helical regions of IL-4 and linked to each other as indicated. Alternative preferred peptides are composed of amino acid sequences derived from the αC-αA-αD helical regions of IL-4 or IL-13 and linked to each other as indicated.

It is apparent that one or more amino acids within the peptide residues A, B or C can be changed by an alternative amino acid, either modified or unmodified, or substituted by one or more amino acids without significantly changing the binding capabilities and/or biological activity. Therefore, any peptide or variant thereof comprising the above structure but having a different amino acid at one or more positions and which is capable of stimulating neuronal axon outgrowth is encompassed by the present invention. The neuronal axon outgrowth as defined herein can be measured by the cortex growth assay described herein.

In the context of the present invention, the standard one-letter code for amino acid residues as well as the standard three-letter code are applied. Abbreviations for amino acids are in accordance with the recommendations in the IUPAC-IUB Joint Commission on Biochemical Nomenclature Eur. J. Biochem, 1984, vol. 184, pp 9-37. Throughout the description and claims either the three letter code or the one letter code for natural amino acids are used. Where the L or D form has not been specified it is to be understood that the amino acid in question has the natural L form, cf. Pure & Appl. Chem. Vol. (56(5) pp 595-624 (1984) or the D form, so that the peptides formed may be constituted of amino acids of L form, D form, or a sequence of mixed L forms and D forms.

The C-terminal amino acid of a peptide usually exists as a free carboxylic acid, which may also be specified as "-OH". However, the C-terminal amino acid of a peptide for use according to the present invention may also be an amidated derivative, which is specified as "-NH-2". Where nothing else is stated, the N-terminal amino acid of a polypeptide comprises a free amino-group, sometimes also specified as "H-". A peptide, fragment or variant thereof according to the present invention can also comprise one or several unnatural or modified natural amino acids.

Preferred compounds of the present invention are peptides which are IL-4 derivatives or IL-13 derivatives that are able to specifically bind to the IL-4 receptor type I and II by including the amino acids within the α-helices αA, αB, αC and αD that are required for binding to IL-4Rα combined with the amino acids that are required for binding to the co-chains IL-13Rα1 or common γ chain.

In order to obtain the compounds of the present invention, the inventors coupled the most important receptor-binding amino acids and linked the co-receptor chains to each other. The amino acid stretches were selected based upon their binding to the IL-4Ra chain and either of the IL-13Rα1 or common γ chain, thereby linking the chains to form either IL-4R type I or type II. All compounds are based on the same principle of receptor chain linking and all IL4- and IL-13 derivatives have similar mechanisms. The selected amino acids derived from the αA, αB, αC αD helical region of human or animal IL-4 or IL-13 are the most essential amino acids required for binding of the inventive compound to IL-4Rα or IL-13Rα.

The present invention covers any peptide or polypeptide, both in modified or un-modified form, that is based on a mammalian amino acid sequence of one or more of the αA, αB, αC αD helical regions of mammalian (e.g. human or murine) IL-4 or IL-13. The compound of the present invention can comprise one or more of said peptides, optionally bridged by structurally neutral amino acids. Specifically, the present invention covers the combination of amino acid stretches found in the αA, αB, αC αD helical regions of IL-4 or IL-13 in mammals, which are required for binding to IL-4Ra and IL-13Rα1 or common γ chain. Although the present invention is exemplified herein using human and murine IL-4 and IL-13, the invention also covers homologous amino acids and amino acid stretches found in other species in which the same principles of IL-4/ IL-4Rα and IL-13/ IL-13Rα interaction apply.

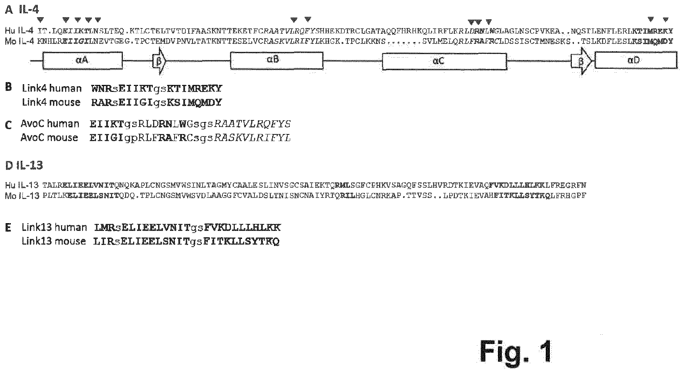

Preferably, the amino acid sequences of the αA, αB, αC αD helical regions are derived from mammalian IL-4. The amino acid sequence of human and murine IL-4 covering the four α A to α D alpha helices is shown in Fig. 1A. In an alternative embodiment, the peptides of the present invention are derived from selected αA, αB, αC αD helical regions of mammalian IL-13. The amino acid sequence of human and murine IL-13 covering the four α A to α D alpha helices is shown in Fig. 1D.

The arrangement of the different helical sections for generation of the compounds of the invention is shown. Given the general structure of the IL-4- or IL-13-derived peptides of the present invention, A - L1 - B - L2 - C, it is preferred that A corresponds to the αC helix of IL-4 or IL-13 followed by a first linker sequence which is connected to a selected amino acid sequence of the αA helix of IL-4 or IL-13 followed by a second linker sequence which is connected to a selected amino acid sequence derived from the αD helix of IL-4 or IL-13. The linker sequences are necessary for the 3-D structure of the peptides of the invention.

In an alternative embodiment, A corresponds to selected amino acids derived from the αA helix of IL-4 followed by the first linker sequence which is connected to a selected amino acid sequence of the αC helix of IL-4 followed by a second linker sequence which is connected to a selected amino acid sequence of the αB helix of IL-4. Preferably, the selected amino acids derived from one or more, preferably from at least three amino acid stretches in the αA, αB, αC αD helical regions, are contiguous amino acids that can be found in said region of a selected species, preferably IL-4 or IL-13 of a human or other mammalian species.

Preferred compounds of the present invention are peptides, wherein
A corresponds to the amino acids WNR, RAR, LMR, LIR, EIIKT, or EIIGI
B corresponds to the amino acids EIIKT, EIIGI, ELIEELVNIT, ELIEELSNIT, RLDRNLWG, or RLFRAFRC
C corresponds to the amino acids KTIMREKY, FVKDLLLHLKK, RAATVLRQFYS, KSIMQMDY, FITKLlSYTKQ, or RASKVLRIFYL,
or a variant thereof having a different amino acid at one position, wherein said peptide is capable of stimulating neuronal axon outgrowth.

A variant according to the present invention may comprise an amino acid sequence which has at least 70% positive amino acid matches with a selected sequence of the αA, αB, αC αD helical regions, such as 71-80% positive amino acid matches, preferably at least 80%, more preferably at least 90% positive amino acid matches, for example 91 to 99% positive amino acid matches with a selected sequence of the oA, αB, αC αD helical regions. A positive amino acid match is defined as the presence at the same position in two compared sequences of amino acid residues which has similar physical, biological and/or chemical properties. Preferred positive amino acid matches that could be used for substitutions are K to R, E to D, L to M, Q to E, I to V, I to L, A to S, Y to W, K to Q, S to T, N to S and Q to R. It is required that a peptide or variant or the present invention has the capability of axon outgrowth which can be measured by an axon outgrowth assay described herein.

A peptide or variant according to the present invention may also comprise other chemical moieties such as phosphoryl, sulphur, acetyl, or glycosyl moieties. As such, a given peptide sequence may be modified, for example by addition, deletion, substitution or chemical modification of one or more of the amino acid residues. For such modifications, both L-amino acids and D-amino acids may be used. Possible chemical modifications may comprise derivatives such as sugars or esters, for example methyl or acetyl esters, or polyethylene glycol modifications. Furthermore, an amine group of the peptide may be converted to an amide, which comprises a fatty acid. A peptide or variant of the invention can also be modified by biotin at it C-terminus or by a His-Tag at its N-terminus.

According to the present invention, variants of the amino acid sequences may comprise one or more conservative amino acid substitutions, which are independent of one another, wherein (i) at least one glycine (Gly) of said variant is substituted with an amino acid selected from the group of amino acids consisting of Ala, Val, Leu, and lie
(ii) at least one alanine (Ala) of said variant is substituted with an amino acid selected from the group of amino acids consisting of Gly, Val, Leu,
(iii) at least one valine (Val) of said variant is substituted with an amino acid selected from the group of amino acids consisting of Gly, Ala, Leu, and Ile,
(iv) at least one leucine (Leu) of said variant is substituted with an amino acid selected from the group of amino acids consisting of Gly, Ala, Val, and IIe,
(v) at least one isoleucine (IIe) of said variant is substituted with an amino acid selected from the group of amino acids consisting of Gly, Ala, Val and Leu,
(vi) at least one aspartic acids (Asp) of said variant is substituted with an amino acid selected from the group of amino acids consisting of Glu, Asn, and Gin,
(vii) at least one aspargine (Asn) of said variant is substituted with an amino acid selected from the group of amino acids consisting of Asp, Glu, and Gln,
(viii) at least one glutamine (GIn) of said variant is substituted with an amino acid selected from the group of amino acids consisting of Asp, Glu, and Asn,
(ix) at least one phenylalanine (Phe) of said variant is substituted with an amino acid selected from the group of amino acids consisting of Tyr, Trp, His, Pro, preferably selected from the group of amino acids consisting of Tyr and Trp,
(x) at least one tyrosine (Tyr) of said variant is substituted with an amino acid selected from the group of amino acids consisting of Phe, Trp, His, Pro, preferably an amino acid selected from the group of amino acids consisting of Phe and Trp,
(xi) at least one arginine (Arg) of said variant is substituted with an amino acid selected from the group of amino acids consisting of Lys and His,
(xii) at least one lysine (Lys) of said variant is substituted with an amino acid selected from the group of amino acids consisting of Arg and His
(xiii) at least one proline (Pro) of said variant is substituted with an amino acid selected from the group of amino acids consisting of Phe, Tyr, Trp, and His,
(xiv) at least one cysteine (Cys) of said variant is substituted with an amino acid selected from the group of amino acids consisting of Asp, Glu, Lys, Arg, His, Asn, GIn, Ser, Thr, and Tyr.

It thus follows from the above that the same variant of a peptide or a fragment thereof may comprise more than one conservative or homologous amino acid substitution from more than one group of conservative amino acids as defined herein above.

Conservative amino acid substitutions may be introduced in any position of a peptide or variant of the present invention. It may however also be desirable to introduce non-conservative substitutions, particularly, but not limited to, a non-conservative substitution at one or more positions. Substitution of amino acids may be made based upon their hydrophobicity and hydrophilicity values and the relative similarity of the amino acid side-chain substituents such as size and charge.

The groups of conservative amino acids are preferably the following, depending on their known chemical properties:
(i) A, G; (ii) Q, N, S, T; (iii) E, D; (iv) Q, N, S, T; (v) H, K, R; (vi) L, P, I, V, M, F, Y, W

Preferred peptides of the present invention are derived from human IL-4, wherein
A corresponds to the amino acids WNR,
B corresponds to the amino acids EIIKT,
C corresponds to the amino acids KTIMREKY,
or a variant thereof having a different amino acid at one position, wherein said peptide is capable of stimulating neuronal axon outgrowth.

Preferred peptides of the present invention are peptides derived from human IL-13, wherein
A corresponds to the amino acids LMR,
B corresponds to the amino acids ELIEELVNIT,
C corresponds to the amino acids FVKDLLLHLKK,
or a variant thereof having a different amino acid at one position, wherein said peptide is capable of stimulating neuronal axon outgrowth.

Preferred peptides of the present invention are IL-4 derivatives, wherein
A corresponds to the amino acids EIIKT,
B corresponds to the amino acids RLDRNLWG,
C corresponds to the amino acids RAATVLRQFYS,
or a variant thereof having a different amino acid at one position, wherein said peptide is capable of stimulating neuronal axon outgrowth.

The biological activity of a peptide of the present invention is defined by its capability to bind to IL-4R type I or II, wherein the binding of the peptide or variant of the invention results in the desired neuroprotective and neuroregenerative effects. These biological effects can be measured by *in vitro* or *in vivo* model systems such as experimental autoimmune encephalomyelitis (EAE) or other mouse models for multiple sclerosis (MS). In addition, biological activity of a peptide or variant of the invention can be measured by a cortex growth assay.

In order to connect the amino acid stretches A, B, C of a compound comprising the above formula, linker sequences are used that are preferably comprised of one or more amino acids, preferably the linker consists of 1 to 10, more preferably 1 to 5 amino acids. A first linker sequence connects the amino acids of A and B, whereas a second linker sequence connects the amino acids of B and C in the above formula. Preferably, the first linker sequence (L1) and/or the second linker sequence (L2) correspond to S, GS, SGS, P, GP or PGP. It is apparent for the person skilled in the art that also other amino acids or linkers can be used in order to connect the amino acid stretches derived from the α helical regions of IL-4 and IL-13. Any linker that is suitable for this purpose falls within the definition of the present invention. The linker is necessary to combine the selected alpha helical regions to each other and to mimic the 3-D structure of the receptor binding region.

In a preferred embodiment, the peptide of the present invention is a human IL-4 derivative comprising an amino acid sequence WNRSEIIKTGSKTIMREKY (SEQ ID NO: 1), or a variant thereof having a different amino acid at one or more positions, wherein said peptide is capable of stimulating neuronal axon outgrowth. This peptide is composed of the αC, αA and αD helical regions linked together by a first and second linker acid sequence consisting of one or more amino acids.

In an alternative embodiment, the peptide of the present invention is a human IL-13 derivative comprising an amino acid sequence LMRSELIEELVNITGSFVKDLLLHLKK (SEQ ID NO: 2), or a variant thereof having a different amino acid at one or more positions, wherein said peptide is capable of stimulating neuronal axon outgrowth. This peptide is composed of the αC, αA and αD helical regions linked together by a first and second linker acid sequence consisting of one or more amino acids.

In an alternative embodiment, the peptide of the present invention is a human IL-4 derivative comprising an amino acid sequence EIIKTGSRLDRNLWGSGSRAATVLRQFYS (SEQ ID NO: 3), or a variant thereof having a different amino acid at one or more positions, wherein said peptide is capable of stimulating neuronal axon outgrowth. This peptide is composed of the αA, αC, and αB helical regions linked together by a first and second linker acid sequence consisting of one or more amino acids.

In an alternative embodiment, the peptide of the present invention is a murine IL-4 derivative comprising an amino acid sequence RARSEIIGIGSKSIMQMDY (SEQ ID NO: 4), or a variant thereof having a different amino acid at one or more positions, wherein said peptide is capable of stimulating neuronal axon outgrowth. This peptide is composed of the αC, αA and αD helical regions linked together by a first and second linker acid sequence consisting of one or more amino acids.

In an alternative embodiment, the peptide of the present invention is a murine IL-13 derivative comprising an amino acid sequence LIRSELIEELSNITGSFITKLLSYTKQ (SEQ ID NO: 5), or a variant thereof having a different amino acid at one or more positions, wherein said peptide is capable of stimulating neuronal axon outgrowth. This peptide is composed of the αC, αA and αD helical regions linked together by a first and second linker acid sequence consisting of one or more amino acids.

In an alternative embodiment, the peptide of the present invention is a murine IL-4 derivative comprising an amino acid sequence EIIGIGPRLFRAFRCSGSRASKVLRIFYL (SEQ ID NO: 6), or a variant thereof having a different amino acid at one or more positions, wherein said peptide is capable of stimulating neuronal axon outgrowth. This peptide is composed of the αA, αC, and αB helical regions linked together by a first and second linker acid sequence consisting of one or more amino acids.

The IL-4 and IL-13 derivatives of the present invention are able to act on neurons in the same way as IL-4 but without the side effects of affecting lymphocyte populations or myeloid cells. It is also shown by the present invention that IL-4 and IL-13 derivatives are not toxic and exhibit beneficial effects on autoimmune encephalomyelitis *in vivo* as shown by the EAE model. The compounds also show beneficial effects on axon growth and neuronal signalling and have only minimal effects on inflammatory cells. As shown herein, the IL-4 and IL-13 derivatives of the present invention led to a significant amelioration of the clinical score similar to native IL-4.

As such, the present invention also covers a pharmaceutical composition comprising at least one compound of the present invention, and a pharmaceutically suitable carrier, vehicle or agent. Preferably such a carrier, vehicle or agent is suitable for administration of a pharmaceutically active ingredient, for example transmucosal administration via the nasal mucosa. A vehicle as used in the pharmaceutical composition may comprise solvents, cosolvents, enhancers, pH buffering agents, antioxidants, additives or the like. The various components of the vehicle are non-toxic and do not interact with other components of the total composition in a deleterious manner.

Preferably, the compound of the present invention is contained in a therapeutically effective amount in the pharmaceutical composition, i.e. in a concentration that is non-toxic, but sufficient to act as a drug or active agent to provide a desired therapeutic effect. For example, one or more doses of a compound of the present invention will be effective in treatment of a neuroinflammatory or neurodegenerative disorder, or neuropathies or traumatic nervous system injuries. The compounds of the present invention act on neurons without effecting T-cells or myeloid cells.

As exemplified by the *in vitro* and *in vivo* data described herein, the compounds of the present invention are suitable for use in the treatment or prevention of a neuroinflammatory or neurodegenerative disorder. Preferred neuroinflammatory or neurodegenerative disorders include all forms of multiple sclerosis (MS), neuromyelitis optica (NMO), Parkinson's disease,

Alzheimer's disease or other forms of dementia, amyotrophic lateral sclerosis (ALS) and Huntington's disease.

The compounds of the present invention are also suitable for use in the treatment or prevention of neuropathies or traumatic nervous system injuries. Preferred neuropathies are Charcot Marie Tooth disease, Guillain Barre Syndrome, Chronic inflammatory demyelinating polyneuropathy and diabetic neuropathies.

Preferred traumatic nervous system injuries are spinal cord injuries, traumatic brain injuries, stroke, peripheral nerve injuries.

The pharmaceutical applicability is exemplified herein by lumbar puncture (intrathecal injection), nasal application and systemic injection.

The present invention is further illustrated in the following examples:

### Design of IL-4 derivatives and IL-13 derivatives

The IL-4 derivatives and IL-13 derivatives of the invention are compounds that are defined by the structure A - L1 - B - L2 - C. Following this structure, the inventors tested various IL-4 derivatives that they derived from different a helical regions of human and murine IL-4. These newly generated IL-4 derivatives are named as "Link4" and "AvoC" herein.

The inventors also tested various IL-13 derivatives that are derived from the α helical regions of human and murine IL-13 and follow the above formula. These newly generated IL-13 derivatives are named "Link13" herein.

In Fig. 1, the design of the derivatives Link4 and Link13 is described. Based on the known 3-D structure of IL-4 and the IL-4 receptor, the inventors combined the amino acid stretches that are required to bind to IL-4R. The inventors then identified which amino acids were located on the surface of the IL-4 protein to decide which combinations of the αA to αD helices to make. A part of the αC helix was turned around and connected with one linking amino acid to a part of the αA helix which was bound with two linking amino acids to a part of the αD helix. The receptor-binding amino acids have been identified and coupled, which yielded IL-4 and IL-13 derivatives in which the co-receptor chains were linked to each other. The derivatives of IL-4 and IL-13 were designed in a similar manner based upon the sequence homology of IL-4 and IL-13.

The IL-4 derivative AvoC was designed based upon another binding principle of IL-4 to its receptor. The binding sequence of IL-4 is defined by so-called avocado clusters in which a core is surrounded by hydrophobic residues (T. D. Mueller, J. L. Zhang, W. Sebald, A. Duschl, Structure, binding and antagonists in the IL-4/IL-13 receptor system, Biochimica Biophysica Acta 1592, 237-250 (2002)). Stretches of amino acids were arranged in such a way to mimic the surface structure of IL-4. Also this derivative is likely to link the two receptor chains, since the sequence derived from the αA helix binds to both receptor chains.

Fig. 1A shows the amino acid sequences of human and murine IL-4 aligned with the location of the α-helices αA, αB, αC, αD and the β-linkers. Black arrowheads indicate binding sites to the IL-4Ra chain, grey arrowheads point to binding sites to the co-chains, either IL-13Rα1 or common γ-chain. The amino acids in bold letters are the amino acids that are used for the construction of the IL-4 derivatives of the present invention as shown in Fig. 1B and 1C.

Fig. 1B shows the IL-4 derivative Link4 with the selected amino acids from the αC, αA and αD helical regions in bold letters.

Fig. 1C shows the IL-4 derivative AvoC with the selected amino acids from the αA, αC, and αB helical regions in bold letters.

Fig. 1D shows the aligned amino acid sequences of human and murine IL-13 comprising the α-helices αA, αB, αC, αD. The amino acids used to design the IL-13 derivative Link13 of the present invention are depicted.

Fig. 1E shows the IL-13 derivative Link13 with the selected amino acids from the αC, αA and αD helical regions in bold letters.

Fig. 2 shows a toxicity assay for mouse embryonic fibroblasts (Met) (Fig. 2A) and hippocampal neurons (HT22) (Fig. 2B). Toxicity was checked by subjecting mouse embryonic fibroblasts (Mef) and the hippocampal cell line HT22 to a CellTiter-blue cell viability assay (ctb). Link4 and AvoC, applied in increasing concentrations, had no toxic effects on fibroblasts (Fig. 2A)or hippocampal neurons (Fig. 2B).

Fig. 3 shows the *in vivo* effects of the IL-4 derivative Link4. Treatment of mice during the chronic phase of EAE (grey bar) via lumbar (A) intrathecal injection or (B) nasal application showed a significant improvement in the clinical score of IL-4- and Link4-treated animals, as compared to PBS controls that remained at a high sickness level. Also shown is the lumbar treatment with the Ph8 peptide which had no effect on the disease course.

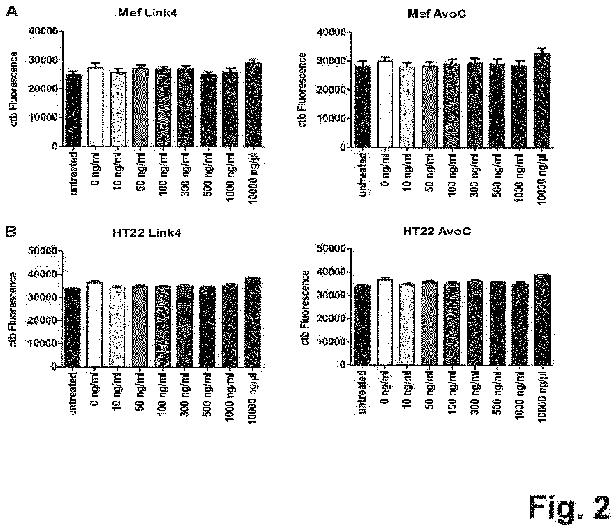

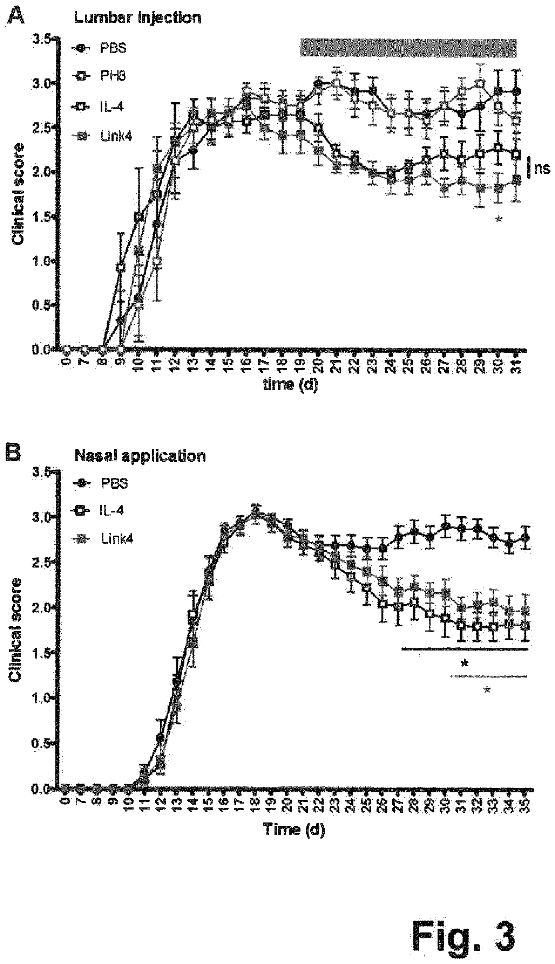

To test whether Link4 has similar effects to IL-4 *in vivo,* two pilot experiments were performed. In the first experiment (Fig. 3A), BI6 mice were subjected to EAE and treated with IL-4, Link4, Ph8 and PBS (n = 6-7 animals per group) via lumbar intrathecal injection directly into the cerebrospinal fluid (CSF) every other day for 14 days, in the chronic phase of the disease starting from day 5 after the first disease peak (grey bar). The treatment with Link4 led to a significant amelioration of the clinical score, similar to IL-4, whereas the known partial agonist Ph8 did not improve clinical signs. For better clinical translation, the treatment was then switched to nasal treatment with IL-4 and Link4 (Fig. 3B; 2 pooled experiments, n = 15-16 per group). Nasally applied treatments can enter the brain through the cribriform plate, where olfactory nerve fibers cross through the bone, resulting in a connection between the roof of the nasal cavity and the brain (C. F. Xiao, F. J. Davis, B. C. Chauhan, K. L. Viola, P. N. Lacor, P. T. Velasco, W. L. Klein, and N. B. Chauhan, Brain transit and ameliorative effects of intranasally delivered anti-amyloid-beta oligomer antibody in 5XFAD mice. J Alzheimers Dis 35, 777-788 (2013)). Again, both IL-4 and Link4 treatments were beneficial.

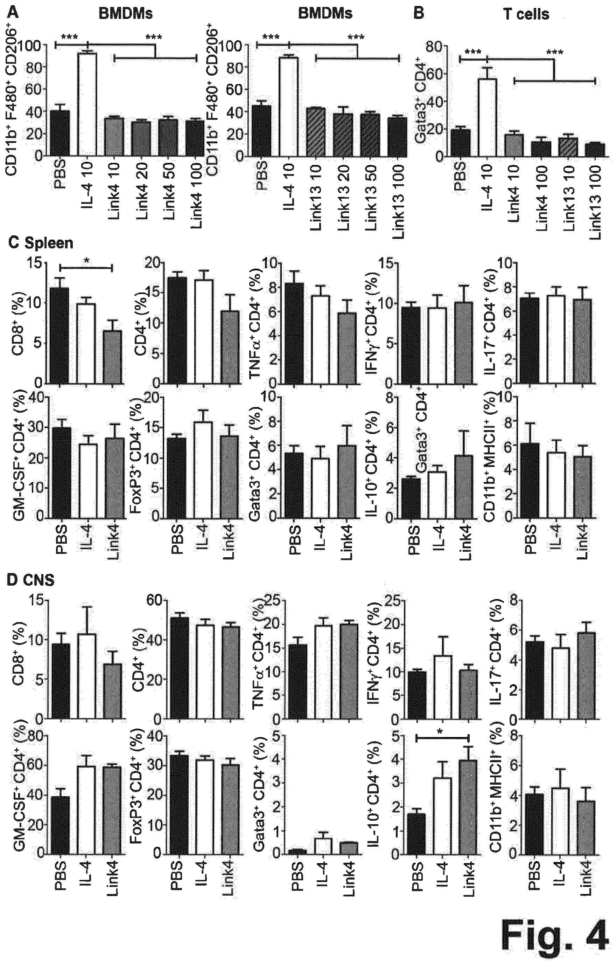

Fig. 4 shows the effects of the inventive IL-4 and IL-13 derivatives on the immune system. A-B: Effects of incubation with IL-4 and different concentrations of Link4 and Link13 on the differentiation of A: CD11b⁺ bone marrow-derived macrophages (BMDMs) into F80⁺ CD206⁺ cells, and B: CD4⁺ T cells into Gata3⁺ cells. C-D: FACS results for different populations of T cells and CD11b⁺ MHCII⁺ monocytes/macrophages/microglia of immune cells isolated from C: spleen and D: central nervous system (CNS) of EAE mice treated with PBS, IL-4 or Link4.

In order to investigate whether Link4 and Link13 are able to modulate immune cells, *in vitro* assays were performed on bone marrow-derived macrophages (BDMBs) and on naïve T cells. Treatment of BDMBs with IL-4 increased the expression of the differentiation markers F80 and CD206 on CD11b⁺ BMDMs. This differentiation did not occur in response to Link4 or Link13 (Fig. 4A). The expected differentiation into Gata3⁺ CD4⁺ T cells observed with IL-4 again did not take place when cells were incubated with Link4, or Link13 (Fig. 4B). To rule out that the Link-analogues were needed in a higher concentration than IL-4, the assays were performed with increasing amounts, which did not make a difference.

Subsequently, the effects of IL-4 and Link4 on immune cells were analysed when applied intranasally, similarly as in Fig. 3B. Since nasally applied treatments can enter the periphery (lungs, blood stream) it was of utmost importance to check whether the substances affected the lymphocyte populations in the spleen. A reduction was found of CD8⁺ cytotoxic T-cells for Link4, whereas all CD4⁺ T helper cell types were unchanged. The CD11b+ MHCII+ population of monocytes/macrophages was also not changed. For the populations of lymphocytes entering the brain and spinal cord (taken together as CNS, central nervous system), only a significant rise in IL-10⁺ cells was observed.

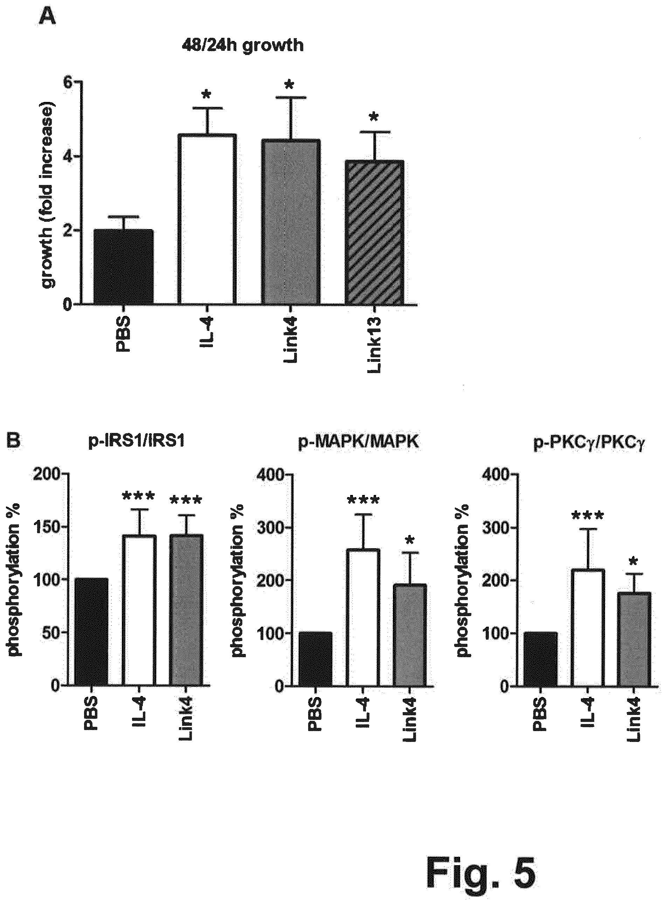

Fig. 5 shows the effects of the IL-4 and IL-13 derivatives of the invention on neurons. A: Cortical outgrowth in response to 50 ng/ml IL-4, Link4, or Link13, compared to PBS control. The increase in growth at 48h was calculated over the basal (untreated) growth at 24h. B: Effects on phosphorylation of signalling molecules after 10 min treatment of dissociated cortical neurons with IL-4, Link4 or PBS. Link4 showed the same neuronal signalling effects as IL-4

### Materials and Methods

### Experimental autoimmune encephalomyelitis (EAE)

For active EAE, female 9-10-weeks-old C57BI6 mice were immunized as previously described (M. Paterka, J. O. Voss, J. Werr, E. Reuter, S. Franck, T. Leuenberger, J. Herz, H. Radbruch, T. Bopp, V. Siffrin, and F. Zipp, Dendritic cells tip the balance towards induction of regulatory T cells upon priming in experimental autoimmune encephalomyelitis. J Autoimmun 67, 108-114 (2017)) by subcutaneous injection of 200 µg myelin oligodendrocyte protein 35-55 (MOG₃₅₋₅₅) mixed with complete Freund adjuvant (CFA). Following the MOG₃₅₋₅₅ immunization 400 ng pertussis toxin (PTX) was administered intraperitoneally at the day of immunizaton and after 24 h. Clinical signs were scored using the following parameters:

| **Score** | **Signs of EAE** |
|---|---|
| 0 | no detectable signs |
| 0.5 | tail weakness |
| 1 | complete tail paralysis |
| 2 | partial hind limb paralysis |
| 2.5 | unilateral complete hind limb paralysis |
| 3 | complete bilateral limb paralysis |
| 3.5 | complete hind limb paralysis and parti forelimb paralysis |
| 4 | total paralysis of forelimbs and hind limbs |
| 5 | death |

Treatment with rIL-4 (1µg, Peprotech), Ph8 (1 µg, custom synthesized, Schafer N), Link4 (1 µg, custom synthesized, Schafer N) or vehicle (PBS) was performed during the chronic phase of the disease models by lumbar intrathecal injection (R. Lu and A. Schmidtko, Direct intrathecal drug delivery in mice for detecting in vivo effects of cGMP on pain processing. Methods Mol Biol 1020, 215-221 (2013)) or nasal application of IL-4 and Link4 was performed according to published procedures (C. F. Xiao, F. J. Davis, B. C. Chauhan, K. L. Viola, P. N. Lacor, P. T. Velasco, W. L. Klein, and N. B. Chauhan, Brain transit and ameliorative effects of intranasally delivered anti-amyloid-beta oligomer antibody in 5XFAD mice. J Alzheimers Dis 35, 777-788 (2013)). Mice were pre-trained to avoid stress and held at a 45 degree angle to apply IL-4 solution (1 µg, Peprotech) to the nostrils using a pipette tip.

### Flow cytometry (FACS)

CNS and spleens were removed from EAE mice treated with nasal IL-4, Link4 or PBS at d35 and immune cells were isolated as previously described (M. Paterka, J. O. Voss, J. Werr, E. Reuter, S. Franck, T. Leuenberger, J. Herz, H. Radbruch, T. Bopp, V. Siffrin, and F. Zipp, Dendritic cells tip the balance towards induction of regulatory T cells upon priming in experimental autoimmune encephalomyelitis. J Autoimmun 67, 108-114 (2017)). For *in vitro* stimulation of T cells, anti-CD3 (145-2C11) and anti-CD28 (37.51) were used. The following antibodies were used for flow cytometry with the BD FACSCanto II (BD Bioscience): anti-CD4 PEcy7 (RM4-5), anti-CD8 FITC (53-6.7), anti-CD3APC (145-2c11), anti-CD11b bio (M1/70), anti-MHCII PE (AF6-120.1), anti-CD11b V450 (HL311B), anti-CD45 AF605 (30-F11), anti-GM-CSF PE (MP1-22E9), anti-IL17 APC (eBio17B7), anti-TNFα AF700 (MP6-XT22), anti-IFN-γV450 (XMG1.2), anti-GATA-3 PE (TWAZ), anti-FOXP3 PEcy7(FZK-16s), anti-IL-10 APC (JES5-16E3), anti-CD4 V450 (RM4-5). All antibodies were purchased from eBioscience or Biolegend.

### Isolation of murine lymphocytes (CD4+CD62L+)

For the isolation of T lymphocytes 5-8 week old C57BL/6 mice were sacrificed by cervical dislocation and the spleens and lymph nodes (LN) were isolated and rubbed trough a 100 µm cell strainer into a 50 ml tube with washing medium (5% FCS, 1% P/S,1 %HEPES in PBS). After washing, the cells were centrifuged for 5 min at 550 g at 4°C. To remove the red blood cells the supernatant was re-suspended in lysis buffer followed by centrifugation (5 min, 550 g, 4°C). The cells were resuspended in MACS buffer and magnetic lymphocyte sorting was performed on ice, using the MidiMACS and QuadroMACS Seperators. A CD4 untouched and CD8 touched cell sort was performed a washing step in ml MACS buffer (5 min, 550 g, 4°C).

Cells were incubated with CD4 T cell biotin antibody cocktail (Miltenyi Biotec) in MACS buffer for 5 min at 4°C. Subsequently, anti-biotin microbeads and CD8 microbeads in MACS buffer was added. CD8 microbeads were added to reduce the possible contamination of CD8⁺ lymphocytes. After 10 min of incubation at 4°C the cells were washed again and resuspended in MACS buffer. MACS columns were pre equilibrated and topped with pre-separation filters (30 µm). A maximum of 300 × 10⁶ cells were added to each column followed by 3 consecutive washing steps with MACS buffer. The CD3⁺CD4⁺CD8⁻ enriched flow-through was collected for the following CD62L touched sort. After centrifugation (5 min, 550 g, 4°C) CD62L microbeads in MACS buffer were added to the cells and incubated for 15 min at 4°C. After washing the cells were resuspended in MACS buffer and loaded on a new column. The positive labeled CD4⁺CD62L⁺ cells were enriched in the column and collected in MACS buffer. The purity of the magnetic cells sorts was assessed by comparing pre- and post-sort samples by flow cytometry. The cells were stained with CD4-Horizon (1:400), CD3-APC (1:600) and CD62L-APC (1:200). CD4⁺CD62L⁺ purity out of the CD4⁺ cells was usually about >97%.

### Isolation of murine antigen-presenting cells (APCs)

Antigen-presenting cells were isolated by lysis of adult C57BL/6 mice spleen and magnetic immune cell sorting with MidiMACS and QuadroMACS Separators. The cell suspension was washed with MACS buffer and centrifuged (5 min, 550 g, 4°C). Subsequently the cells were resuspended in 95 µl MACS buffer and CD90.2 untouched microbeads and incubated for 15 min at 4°C. After washing with MACS buffer and centrifugation (5 min, 550 g, 4°C) the cells were resuspended in MACS buffer, loaded on the columns and collected in MACS buffer. To stop their proliferation cycle the purified APCs were irradiated at 30 Gy/3000 rad.

### T_{H}2 differentiation

For the initial stimulation the CD4⁺CD62L⁺ cells were co-cultured with antigen-presenting cells (APCs) in a 1:5 ratio in a concentration of 6 million cells in 2 ml mouse medium (10% FCS, 1% P/S, 1% L-glutamine, 0.1% β-mercaptoethanol, 1% HEPES in RPMI buffer) per well on a 24-well plate. For unspecific T cell receptor activation 2 µg/ml anti-CD3 was added to the culture. For differentiation into T_{H}2 cells, IL-4 (10 ng/ml), α-IL-12 (10 µg/ml) and α-tFND (10 µg/ml) were added. At day 3 and 5 the cells were split and plated in fresh medium containing 10 µg/ml IL-2 and 10 ng/ml IL-4. At day 7 cells were stimulated with anti-CD3/anti-CD28 and treated with Brefeldin A to block secretion. After 4 hours incubation, a cytokine check of the different T lymphocyte cultures was performed by flow cytometry using CD4-PECy7 (1:1000) for extracellular staining, Fc-receptor-block (1:100) and IFN-g-Horizon (1:200), TNFa-AF700 (1:200) and IL-10-APC (1:200) for intracellular staining. T_{H}2 cells were additionally stained for intranuclear Gata3-PE (1:100).

### Generation of Bone marrow derived macrophages (BMDMs)

Murine BMDMs were isolated from adult C57BL/6 mice. The tibia and femur bones were flushed with sterile PBS and the bone marrow was collected in washing medium. After filtering the cell suspension trough a 100 µm nylon mesh, the cells were centrifuged (5 min, 550 g, 4°C) and resuspended in mouse medium (10% FCS, 1% P/S, 1% L-glutamine, 0.1% β-mercaptoethanol, 1% HEPES in RPMI buffer). For the *in vitro* generation of BMDMs cells were plated in 6 well-plates and exposed to 20 ng/ml Macrophage stimulating factor (M-CSF) for 4 days. For the activation of the cells, Dexametason (5 × 10⁻⁷M), LPS (10 µg/ml) and IL-4 (10 ng/ml) were added to the culture for the following 3 days.

### CellTiter-blue cell viability assay (ctb)

Mouse embryonic fibroblasts (Mef) (Sigma-Aldrich) and hippocampal HT22 (ThermoScientific) cells were cultured according to the manufacturer's protocols. Ctb assays were performed according to the manufacturer's protocols (Promega).

### Cortex growth assay and dissociated cortical neurons

Neonatal (P1-3) cortex explants were dissected from 250 µm thick vibratome (HM650V, Thermo Fisher) sections from Bregma 0 to -1.5 (motor cortex). Cortical layer V was micro-dissected, plated on poly-D-lysine (0.5 mg/ml) and laminin (1 mg/ml)-coated glass coverslips and grown for 24h in neurobasal medium with 2% horse serum, 2% B27, 1% glutamax and 0.5% penicillin/streptomycin. Explants were treated for 48h with rIL-4 (50 ng/ml), Link4 (50 ng/ml), Link13 (50 ng/ml) or PBS. Axon length was assessed using Adobe Photoshop by measuring the distance of the 40 longest axons, corrected for the initial growth at 24h. Dissociated cortical neurons were prepared as previously described (J. T. Walsh, S. Hendrix, F. Boato, I. Smirnov, J. Zheng, J. R. Lukens, S. Gadani, D. Hechler, G. Golz, K. Rosenberger, T. Kammertons, J. Vogt, C. Vogelaar, V. Siffrin, A. Radjavi, A. Fernandez-Castaneda, A. Gaultier, R. Gold, T. D. Kanneganti, R. Nitsch, F. Zipp, and J. Kipnis, MHCII-independent CD4+ T cells protect injured CNS neurons via IL-4. J Clin Invest 125, 699-714 (2015)). Cortices of embryonic day 18 (E18) mice were incubated with Trypsin/DNase solution and dissociated by trituration. Cells were resuspended in plating medium (1x MEM-Glutamax, 20% glucose, 10% HS, 1x PenStrep), filter-sterilize 600,000 cells per well in a 6-well-plate. At 5h after plating, medium was aspirated, wells were washed 2x with warm PBS and cells were cultured in NB-medium. Cultures were allowed to expand for 3 days, before treatment with 50 ng/ml IL-4, 50 ng/ml Link4 or equivalent volumes of PBS. After 10 min, cells were harvested in lysis buffer and processed for Western blotting. Cell culture reagents were obtained from Fisher Scientific and Sigma. For the signaling experiments, cells were incubated with 50 ng/ml IL-4 (Peprotech) or equivalent volumes of PBS for 10 min.

### Western Blotting

For Western blotting, the following primary antibodies were used: anti-IRS1 (EMD Millipore), anti-phospho-IRS1 (Cell Signaling Technology), anti-MAPK and anti-phospho-MAPK (Cell Signaling Technology), anti-PKCγ (Santa-Cruz), anti-phospho-PKCγ (Biozol). DyLight 800/600-coupled secondary antibodies were used for quantitative analysis of the proteins using the Li-Cor Odyssey FC imaging system (Li-Cor Bioscience). Blots probed with the phospho-antibodies were stripped after visualization, to allow incubation with the total antibodies for parallel detection in the same samples.

### Statistical analysis

Statistical analysis was performed using Graphpad Prism 5 (GraphPad Software Inc). Clinical scores were analyzed using repeated-measures two-way ANOVA with posthoc Bonferroni correction. Data obtained from *in vitro* assays and Western blots were subjected to unpaired t-test or one-way ANOVA with Tukey's test for multiple comparison.

### Amino Acid Sequences of human (hu) and murine (mu) Link4, Link 13, AvoC

The following amino acid sequences were determined for the IL-4 and IL-13 derivatives:
huLink4 (SEQ ID NO 1):
   Trp Asn Arg Ser Glu lie lie Lys Thr Gly Ser Lys Thr lie Met Arg Glu Lys Tyr
huLink13 (SEQ ID NO 2):
huAvoC (SEQ ID NO 3):
muLink4 (SEQ ID NO 4):
   Arg Ala Arg Ser Glu IIe Ile Gly IIe Gly Ser Lys Ser IIe Met Gln
muLink13 (SEQ ID NO 5):
muAvoC (SEQ ID NO 6):

### Sequence Listing:

<110> Universitatsmedizin der Johannes Gutenberg-Universität Mainz
<120> Novel IL-4-/IL-13-derived peptide compounds for the treatment or prevention of neurodegenerative or neuroinflammatory diseases
<130> LINK4
<160> 6
<170> PatentIn version 3.5
<210> 1
   <211> 19
   <212> PRT
   <213> Homo sapiens
<400> 1
   Trp Asn Arg Ser Glu Ile Ile Lys Thr Gly Ser Lys Thr Ile Met Arg
   1 5 10 15
   Glu Lys Tyr
<210> 2
   <211> 27
   <212> PRT
   <213> Homo sapiens
<400> 2
   Leu Met Arg Ser Glu Leu Ile Glu Glu Leu Val Asn Ile Thr Gly Ser
   1 5 10 15
   Phe Val Lys Asp Leu Leu Leu His Leu Lys Lys
   20 25
<210> 3
   <211> 29
   <212> PRT
   <213> Homo sapiens
<400> 3
   Glu Ile Ile Lys Thr Gly Ser Arg Leu Asp Arg Asn Leu Trp Gly Ser
   1 5 10 15
   Gly Ser Arg Ala Ala Thr Val Leu Arg Gln Phe Tyr Ser
   20 25
<210> 4
   <211> 19
   <212> PRT
   <213> Mus musculus
<400> 4
   Arg Ala Arg Ser Glu Ile Ile Gly Ile Gly Ser Lys Ser Ile Met Gln
   1 5 10 15
   Met Asp Tyr
<210> 5
   <211> 27
   <212> PRT
   <213> Mus musculus
<400> 5
   Leu Ile Arg Ser Glu Leu Ile Glu Glu Leu Ser Asn Ile Thr Gly Ser
   1 5 10 15
   Phe Ile Thr Lys Leu Leu Ser Tyr Thr Lys Gln
   20 25
<210> 6
   <211> 29
   <212> PRT
   <213> Mus musculus
<400> 6
   Glu Ile Ile Gly Ile Gly Pro Arg Leu Phe Arg Ala Phe Arg Cys Ser
   1 5 10 15
   Gly Ser Arg Ala Ser Lys Val Leu Arg Ile Phe Tyr Leu
   20 25

## Claims

1. A compound consisting of one or more peptides consisting of the following general structure
A - L1 - B - L2 - C
wherein
A corresponds to a first amino acid sequence that is derived from αA or αC alpha helical region of human IL-4 or IL-13,
B corresponds to a second amino acid sequence that is derived from αA or αC alpha helical region of human IL-4 or IL-13,
C corresponds to a third amino acid sequence that is derived from αD or αB alpha helical region of human IL-4, or αD alpha helical region of human IL-13,
L1 and L2 correspond to 1 to 10 linking amino acids,
wherein
A corresponds to the amino acids WNR,
B corresponds to the amino acids EIIKT,
C corresponds to the amino acids KTIMREKY,
or
A corresponds to the amino acids LMR,
B corresponds to the amino acids ELIEELVNIT,
C corresponds to the amino acids FVKDLLLHLKK,
or
A corresponds to the amino acids EIIKT,
B corresponds to the amino acids RLDRNLWG,
C corresponds to the amino acids RAATVLRQFYS,
wherein said peptide is capable of stimulating neuronal axon outgrowth and has no side effect on lymphocytes or bone marrow-derived macrophages.

2. A compound consisting of one or more peptides consisting of the following general structure
A - L1 - B - L2 - C
wherein
A corresponds to a first amino acid sequence that is derived from αA or αC alpha helical region of animal IL-4 or IL-13,
B corresponds to a second amino acid sequence that is derived from αA or αC alpha helical region of animal IL-4 or IL-13,
C corresponds to a third amino acid sequence that is derived from αD or αB alpha helical region of animal IL-4, or αD alpha helical region of animal IL-13,
L1 and L2 correspond to 1 to 10 linking amino acids,
wherein
A corresponds to the amino acids RAR,
B corresponds to the amino acids EIIGI,
C corresponds to the amino acids KSIMQMDY,
or
A corresponds to the amino acids LIR,
B corresponds to the amino acids ELIEELSNIT,
C corresponds to the amino acids FITKLISYTKQ,
or
A corresponds to the amino acids EIIGI
B corresponds to the amino acids RLFRAFRC,
C corresponds to the amino acids RASKVLRIFYL,
wherein said peptide is capable of stimulating neuronal axon outgrowth and has no side effect on lymphocytes or bone marrow-derived macrophages.

3. The compound according to claim 1 or claim 2, wherein L1 and/or L2 correspond(s) to S, GS, SGS, P, GP or PGP.

4. The compound according to claim 1, wherein the peptide is a human IL-4 derivative comprising an amino acid sequence WNRSEIIKTGSKTIMREKY (SEQ ID NO: 1), or a variant thereof having a different amino acid at one position, wherein said peptide is capable of stimulating neuronal axon outgrowth.

5. The compound according to claim 1, wherein the peptide is a human IL-13 derivative comprising an amino acid sequence LMRSELIEELVNITGSFVKDLLLHLKK (SEQ ID NO: 2), or a variant thereof having a different amino acid at one position, wherein said peptide is capable of stimulating neuronal axon outgrowth.

6. The compound according to claim 1, wherein the peptide is a human IL-4 derivative comprising an amino acid sequence EIIKTGSRLDRNLWGSGSRAATVLRQFYS (SEQ ID NO: 3), or a variant thereof having a different amino acid at one position, wherein said peptide is capable of stimulating neuronal axon outgrowth.

7. The compound according to claim 2, wherein the peptide is a murine IL-4 derivative comprising an amino acid sequence RARSEIIGIGSKSIMQMDY (SEQ ID NO: 4), or a variant thereof having a different amino acid at one position, wherein said peptide is capable of stimulating neuronal axon outgrowth.

8. The compound according to claim 2, wherein the peptide is a murine IL-13 derivative comprising an amino acid sequence LIRSELIEELSNITGSFITKLLSYTKQ (SEQ ID NO: 5), or a variant thereof having a different amino acid at one position, wherein said peptide is capable of stimulating neuronal axon outgrowth.

9. The compound according to claim 2, wherein the peptide is a murine IL-4 derivative comprising an amino acid sequence EIIGIGPRLFRAFRCSGSRASKVLRIFYL (SEQ ID NO: 6), or a variant thereof having a different amino acid at one position, wherein said peptide is capable of stimulating neuronal axon outgrowth.

10. A pharmaceutical composition comprising at least one compound according to any one of claims 1 to 9 and a pharmaceutically suitable carrier, vehicle or agent.

11. A compound according to any one of claims 1 to 9 for use in the treatment or prevention of a neuroinflammatory or neurodegenerative disorder.

12. A compound according to any one of claims 1 to 9 for use in the treatment or prevention of neuropathies or traumatic nervous system injuries.

## Patentansprüche

1. Verbindung, bestehend aus einem oder mehreren Peptiden, welche die folgende allgemeine Struktur aufweist
A - L1 - B - L2 - C
wobei
A einer ersten Aminosäuresequenz entspricht, die von der αA- oder αC-alpha-helikalen Region von humanem IL-4 oder IL-13 abgeleitet ist,
B einer zweiten Aminosäuresequenz entspricht, die von der αA- oder αC-alpha - helikalen Region von humanem IL-4 oder IL-13 abgeleitet ist,
C einer dritten Aminosäuresequenz entspricht, die von der αD oder αB-alpha-helikalen Region von humanem IL-4, oder der αD-alpha-helikalen Region des humanen IL-13 abgeleitet ist,
L1 und L2 entsprechen 1 bis 10 verbindenden Aminosäuren,
wobei
A den Aminosäuren WNR entspricht,
B den Aminosäuren EIIKT entspricht,
C den Aminosäuren KTIMREKY entspricht,
oder
A den Aminosäuren LMR entspricht,
B den Aminosäuren ELIEELVNIT entspricht,
C den Aminosäuren FVKDLLLHLKK entspricht,
oder
A den Aminosäuren EIIKT entspricht,
B den Aminosäuren RLDRNLWG entspricht,
C den Aminosäuren RAATVLRQFYS entspricht,
wobei das Peptid in der Lage ist, neuronales Axonwachstum zu stimulieren und keine Nebenwirkung auf Lymphozyten oder aus dem Knochenmark stammende Makrophagen hat.

2. Verbindung, bestehend aus einem oder mehreren Peptiden, welche die folgende allgemeine Struktur aufweist
A - L1 - B - L2 - C
wobei
A einer ersten Aminosäuresequenz entspricht, die von der αA- oder αC-alpha-helikalen Region von tierischem IL-4 oder IL-13 abgeleitet ist,
B einer zweiten Aminosäuresequenz entspricht, die von der αA- oder αC-alpha-helikalen Region von tierischem IL-4 oder IL-13 abgeleitet ist,
C entspricht einer dritten Aminosäuresequenz, die von der αD oder der αB-alpha-helikalen Region von tierischem IL-4, oder der αD-alpha-helikalen Region von tierischem IL-13 abgeleitet ist,
L1 und L2 entsprechen 1 bis 10 verbindenden Aminosäuren,
wobei
A den Aminosäuren RAR entspricht,
B den Aminosäuren EIIGI entspricht,
C den Aminosäuren KSIMQMDY entspricht,
oder
A den Aminosäuren LIR entspricht,
B den Aminosäuren ELIEELSNIT entspricht,
C den Aminosäuren FITKLISYTKQ, entspricht
oder
A den Aminosäuren EIIGI entspricht
B den Aminosäuren RLFRAFRC entspricht,
C den Aminosäuren RASKVLRIFYL entspricht,
wobei das Peptid in der Lage ist, neuronales Axonwachstum zu stimulieren und keine Nebenwirkung auf Lymphozyten oder aus dem Knochenmark stammende Makrophagen hat.

3. Verbindung nach Anspruch 1 oder Anspruch 2, wobei L1 und/oder L2 S, GS, SGS, P, GP oder PGP entspricht/entsprechen.

4. Verbindung nach Anspruch 1, wobei das Peptid ein humanes IL-4-Derivat ist, umfassend eine Aminosäuresequenz WNRSEIIKTGSKTIMREKY (SEQ ID NO: 1) oder eine Variante davon mit einer anderen Aminosäure an einer Position, wobei das Peptid in der Lage ist, neuronales Axonwachstum zu stimulieren.

5. Verbindung nach Anspruch 1, wobei das Peptid ein humanes IL-13-Derivat ist, umfassend eine Aminosäuresequenz LMRSELIEELVNITGSFVKDLLLHLKK (SEQ ID NO: 2) oder eine Variante davon mit einer anderen Aminosäure an ePosition, wobei das Peptid in der Lage ist, neuronales Axonwachstum zu stimulieren.

6. Verbindung nach Anspruch 1, wobei das Peptid ein humanes IL-4-Derivat ist, umfassend eine Aminosäuresequenz EIIKTGSRLDRNLWGSGSRAATVLRQFYS (SEQ ID NO: 3) oder eine Variante davon mit einer anderen Aminosäure an einer Position, wobei das Peptid in der Lage ist, neuronales Axonwachstum zu stimulieren.

7. Verbindung nach Anspruch 2, wobei das Peptid ein murines IL-4-Derivat ist, umfassend eine Aminosäuresequenz RARSEIIGIGSKSIMQMDY (SEQ ID NO: 4) oder eine Variante davon mit einer anderen Aminosäure an einer Position, wobei das Peptid in der Lage ist, neuronales Axonwachstum zu stimulieren.

8. Verbindung nach Anspruch 2, wobei das Peptid ein murines IL-13-Derivat ist, umfassend eine Aminosäuresequenz LIRSELIEELSNITGSFITKLLSYTKQ (SEQ ID NO: 5) oder eine Variante davon mit einer anderen Aminosäure an einer Position, wobei das Peptid in der Lage ist, neuronales Axonwachstum zu stimulieren.

9. Verbindung nach Anspruch 2, wobei das Peptid ein murines IL-4-Derivat ist, umfassend eine Aminosäuresequenz EIIGIGPRLFRAFRCSGSRASKVLRIFYL (SEQ ID NO: 6) oder eine Variante davon mit einer anderen Aminosäure an einer Position, wobei das Peptid in der Lage ist, neuronales Axonwachstum zu stimulieren.

10. Pharmazeutische Zusammensetzung, umfassend mindestens eine Verbindung nach einem der Ansprüche 1 bis 9 und einen pharmazeutisch geeigneten Träger, Vehikel oder Wirkstoff.

11. Verbindung nach einem der Ansprüche 1 bis 9 zur Verwendung bei der Behandlung oder Prävention einer neuroinflammatorischen oder neurodegenerativen Erkrankung

12. Verbindung nach einem der Ansprüche 1 bis 9 zur Verwendung bei der Behandlung oder Prävention von Neuropathien oder traumatischen Verletzungen des Nervensystems.

## Revendications

1. Composé constitué par un ou plusieurs peptide(s) présentant la structure générale qui suit :
A - L1 - B - L2 - C
dans laquelle :
A correspond à une première séquence d'acides aminés qui est dérivée à partir d'une région en hélice alpha αA ou αC de l'être humain IL-4 ou IL-13 ;
B correspond à une deuxième séquence d'acides aminés qui est dérivée à partir d'une région en hélice alpha αA ou αC de l'être humain IL-4 ou IL-13 ;
C correspond à une troisième séquence d'acides aminés qui est dérivée à partir d'une région en hélice alpha αD ou αB de l'être humain IL-4 ou à partir d'une région en hélice alpha αD de l'être humain IL-13 ;
L1 et L2 correspondent à 1 à 10 acide(s) aminé(s) de liaison ;
dans lequel :
A correspond aux acides aminés WNR ;
B correspond aux acides aminés EIIKT ;
C correspond aux acides aminés KTIMREKY ;
ou
A correspond aux acides aminés LMR ;
B correspond aux acides aminés ELIEELVNIT ;
C correspond aux acides aminés FVKDLLLHLKK ;
ou
A correspond aux acides aminés EIIKT ;
B correspond aux acides aminés RLDRNLWG ;
C correspond aux acides aminés RAATVLRQFYS ;
dans lequel ledit peptide dispose de la capacité de stimuler la croissance d'axones neuronaux et n'a pas d'effets secondaires indésirables sur les lymphocytes ou sur les macrophages issus de la moelle osseuse.

2. Composé constitué par un ou plusieurs peptide(s) présentant la structure générale qui suit :
A - L1 - B - L2 - C
dans laquelle :
A correspond à une première séquence d'acides aminés qui est dérivée à partir d'une région en hélice alpha αA ou αC d'un animal IL-4 ou IL-13 ;
B correspond à une deuxième séquence d'acides aminés qui est dérivée à partir d'une région en hélice alpha αA ou αC d'un animal IL-4 ou IL-13 ;
C correspond à une troisième séquence d'acides aminés qui est dérivée à partir d'une région en hélice alpha αD ou αB d'un animal IL-4 ou à partir d'une région en hélice alpha αD d'un animal IL-13 ;
L1 et L2 correspondent à 1 à 10 acide(s) aminé(s) de liaison ;
dans lequel :
A correspond aux acides aminés RAR ;
B correspond aux acides aminés EIIGI ;
C correspond aux acides aminés KSIMQMDY ;
ou
A correspond aux acides aminés LIR ;
B correspond aux acides aminés ELIEELSNIT ;
C correspond aux acides aminés FITKLISYTKQ ;
ou
A correspond aux acides aminés EIIGI ;
B correspond aux acides aminés RLFRAFRC ;
C correspond aux acides aminés RASKVLRIFYL ;
dans lequel ledit peptide dispose de la capacité de stimuler la croissance des axones neuronaux et n'a pas d'effet secondaire indésirable sur les lymphocytes ou sur les macrophages issus de la moelle osseuse.

3. Composé selon la revendication 1 ou la revendication 2, dans lequel L1 et/ou L2 correspond(ent) à S, GS, SGS, P, GP ou PGP.

4. Composé selon la revendication 1, dans lequel le peptide est un dérivé IL-4 de l'être humain qui comprend une séquence d'acides aminés WNRSEIIKTGSKTIMREKY (SEQ ID NO : 1) ou un variant afférent qui comporte un acide aminé différent au niveau d'une position, dans lequel ledit peptide dispose de la capacité de stimuler la croissance des axones neuronaux.

5. Composé selon la revendication 1, dans lequel le peptide est un dérivé IL-13 de l'être humain qui comprend une séquence d'acides aminés LMRSELIEELVNITGSFVKDLLLHLKK (SEQ ID NO: 2) ou un variant afférent qui comporte un acide aminé différent au niveau d'une position, dans lequel ledit peptide dispose de la capacité de stimuler la croissance des axones neuronaux.

6. Composé selon la revendication 1, dans lequel le peptide est un dérivé IL-4 de l'être humain qui comprend une séquence d'acides aminés EIIKTGSRLDRNLWGSGSRAATVLRQFYS (SEQ ID NO: 3) ou un variant afférent qui comporte un acide aminé différent au niveau d'une position, dans lequel ledit peptide dispose de la capacité de stimuler la croissance des axones neuronaux.

7. Composé selon la revendication 2, dans lequel le peptide est un dérivé IL-4 de la souris qui comprend une séquence d'acides aminés RARSEIIGIGSKSIMQMDY (SEQ ID NO : 4) ou un variant afférent qui comporte un acide aminé différent au niveau d'une position, dans lequel ledit peptide dispose de la capacité de stimuler la croissance des axones neuronaux.

8. Composé selon la revendication 2, dans lequel le peptide est un dérivé IL-13 de la souris qui comprend une séquence d'acides aminés LIRSELIEELSNITGSFITKLLSYTKQ (SEQ ID NO : 5) ou un variant afférent qui comporte un acide aminé différent au niveau d'une position, dans lequel ledit peptide dispose de la capacité de stimuler la croissance des axones neuronaux.

9. Composé selon la revendication 2, dans lequel le peptide est un dérivé IL-4 de la souris qui comprend une séquence d'acides aminés EIIGIGPRLFRAFRCSGSRASKVLRIFYL (SEQ ID NO : 6) ou un variant afférent qui comporte un acide aminé différent au niveau d'une position, dans lequel ledit peptide dispose de la capacité de stimuler la croissance des axones neuronaux.

10. Composition pharmaceutique comprenant au moins un composé selon l'une quelconque des revendications 1 à 9 et un excipient, un vecteur ou un agent pharmaceutiquement approprié.

11. Composé selon l'une quelconque des revendications 1 à 9 pour une utilisation au niveau du traitement ou de la prévention d'une pathologie neuro-inflammatoire ou neurodégénérative.

12. Composé selon l'une quelconque des revendications 1 à 9 pour une utilisation au niveau du traitement ou de la prévention de neuropathies ou de lésions traumatiques du système nerveux.
